# EUROPEAN PATENT SPECIFICATION

(11) **EP 3 353 323 B1**
(45) Date of publication and mention of the grant of the patent: **23.03.2022**
(21) Application number: 16777855.4
(22) Date of filing: 22.09.2016
(51) Int. Cl.: C12Q 1/6825, C12Q 1/6837, C12Q 1/6851

(54) **SYSTEMS AND METHODS FOR ANALYSIS OF NUCLEIC ACIDS**
SYSTEME UND VERFAHREN ZUR ANALYSE VON NUKLEINSÄUREN
SYSTÈMES ET PROCÉDÉS D'ANALYSE D'ACIDES NUCLÉIQUES

(30) Priority: 22.09.2015 US 201562221776 P
(43) Date of publication of application: 01.08.2018
(73) Proprietor: Life Technologies Corporation, Carlsbad, CA 92008 (US)
(72) Inventor: LI, Shifeng, Fremont, California 94538 (US); DONG, Shoulian, Carlsbad, California 92008 (US); STRAUB, Theodore, Carlsbad, California 92008 (US); KEYS, David, Carlsbad, California 92008 (US)
(74) Representative: Ziermann, Oliver
(86) International application number: PCT/US2016/053114
(87) International publication number: WO 2017/053570

(56) References cited:
- EP-A1- 1 542 009
- WO-A1-2008/107014
- WO-A1-2013/158313
- US-A1- 2011 045 466
- US-A1- 2011 159 481
- ERIC SALM ET AL: "Electrical Detection of Nucleic Acid Amplification Using an On-Chip Quasi-Reference Electrode and a PVC REFET", ANALYTICAL CHEMISTRY, vol. 86, no. 14, 15 July 2014 (2014-07-15) , pages 6968-6975, XP055329876, US ISSN: 0003-2700, DOI: 10.1021/ac500897t
- CHRISTOFER TOUMAZOU ET AL: "Simultaneous DNA amplification and detection using a pH-sensing semiconductor system", HHS PUBLIC ACCESS AUTHOR MANUSCRIPT, vol. 10, no. 7, 1 July 2013 (2013-07-01), pages 641-646, XP055329877, GB ISSN: 1548-7091, DOI: 10.1038/nmeth.2520
- TABATA M ET AL: "Electrochemical real-time monitoring of isothermal nucleic acid amplification for quantitative analysis", 2015 TRANSDUCERS - 2015 18TH INTERNATIONAL CONFERENCE ON SOLID-STATE SENSORS, ACTUATORS AND MICROSYSTEMS, TRANSDUCERS 2015 - 2015 TRANSDUCERS - 2015 18TH INTERNATIONAL CONFERENCE ON SOLID-STATE SENSORS, ACTUATORS AND MICROSYSTEMS, TRANSDUCERS 2015 20, 21 June 2015 (2015-06-21), pages 1553-1556, XP002765676, DOI: 10.1109/TRANSDUCERS.2015.7181234

## Description

### CROSS-REFERENCE TO RELATED APPLICATIONS

This application claims the benefit of priority of U.S. Provisional Patent Application No. 62/221,776, filed on September 22, 2015.

### INTRODUCTION

Amplification assays or processes such as polymerase chain reaction (PCR), ribonucleic acid (RNA) amplification, and the like can be used to directly quantify and clonally amplify nucleic acids (including DNA, cDNA, methylated DNA, or RNA). For example, quantitative PCR (qPCR) and digital PCR (dPCR) amplification assays or processes leverage differing methodologies to quantify one or more target molecules. The amplification of target molecules is important to a number of scientific fields, including, but not limited to, genome sequencing, diagnosis of diseases, forensics, GMO detection, genotyping, and other laboratory methodologies that utilize large concentrations of nucleic acid molecules.

Reagents used for PCR amplification may be specific to one or more target molecules. Based on the detection of amplified product in a reaction site or reaction site, the presence of one or more target molecules can be confirmed in a sample or reaction solution. While many uses for PCR amplification have been discovered and implemented over the years, there exists a need to improve the detection using PCR processes of target molecules within samples of organisms and/or viruses.

Many conventional diagnostics techniques rely on complex systems in order to perform detection, including one or more of thermal cycling blocks, temperature control heaters, and delicate electronics and optical equipment. Developing a relatively low-cost and efficient point-of-need (PON) system for performing such PCR diagnostics is challenging. It would be desirable to provide PCR systems capable of being used as a portable PON technology.

WO 2013/158313 A1 describes methods, reagents and products of nucleic acid amplification and/or analysis, amplification that make use of immobilized and/or soluble primers.

The invention has been defined in the appended claims. Objects, features, and/or advantages of various embodiments of the present teaching will be set forth in part in the description which follows, and in part will be obvious from the description, or may be learned by practice of the present disclosure and/or claims. At least some of these objects and advantages may be realized and attained by the elements and combinations particularly pointed out in the appended claims.

It is to be understood that both the foregoing general description and the following detailed description are exemplary and explanatory only and are not restrictive of the claims; rather the claims should be entitled to their full breadth of scope, including equivalents.

### BRIEF DESCRIPTION OF THE DRAWINGS

The present disclosure can be understood from the following detailed description, either alone or together with the accompanying drawings. The drawings are included to provide a further understanding of the present disclosure, and are incorporated in and constitute a part of this specification. The drawings illustrate exemplary embodiments of the present teachings and together with the description serve to explain certain principles and operation.
FIG. 1A schematically illustrates a system for detecting amplification of one or more target molecules according to an embodiment of the present invention.
FIG. 1B illustrates an exemplary workflow method for detecting the presence of one or more target molecules.
FIG. 2 schematically illustrates an exemplary extension phase of a PCR assay.
FIG. 3 is a diagrammatic plan view of a reaction site of a device.
FIG. 4 is a diagrammatic plan view of an embodiment of a reaction device for performing a PCR assay.
FIG. 5 is a block diagrammatic view of a Complementary Metal-Oxide Semiconductor (CMOS) chip based on a column and pixel design according to an embodiment of the present invention.
FIG. 6 is a diagrammatic view of a flow cell for use with a corresponding reaction device according to an embodiment of the present invention.
FIGS. 7A-7C are diagrammatic views of a portion of a reaction device according to embodiments of the present invention.

### DETAILED DESCRIPTION

This description and the accompanying drawings that illustrate exemplary embodiments should not be taken as limiting. Various mechanical, compositional, structural, electrical, and operational changes may be made without departing from the scope of this description and claims, including equivalents. In some instances, well-known structures and techniques have not been shown or described in detail so as not to obscure the disclosure. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment.

The invention has been defined in the appended claims. For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, numerical parameters should at least be construed in light of the number of reported significant digits and by applying ordinary rounding techniques.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

As used herein, the term "biological sample" or "sample" means a material or substance (e.g., in solid and/or liquid form) comprising one or more biological molecules, chemicals, components, and/or compounds of interest to a user, manufacturer, or distributor of the various embodiments of the present invention described or implied herein. A biological sample may be of a known composition (e.g., comprising one or more target molecules and/or one or more calibration molecules) or of an unknown composition (e.g., a biological sample that may or may not contain a target molecule). A biological sample may include, but is not limited to, one or more of a DNA sequence (including cell-free DNA), an RNA sequence, a gene, an oligonucleotide, an amino acid sequence, a protein, a biomarker, or a cell (e.g., circulating tumor cell), or any other suitable target biomolecule. As used herein, an "organism" means an assembly of molecules functioning as a whole that exhibits the properties of life including, for example, a plant, animal, organ, assemblage or system of organs, fungus, or bacterium.

As used herein, the term "sample solution" means a liquid or fluid comprising at least one biological sample.

As used herein the term "target molecule" or "target" means a molecule or molecular sequence (e.g., a DNA sequence, an RNA sequence, a protein, an amino acid sequence, a gene, a single nucleotide polymorphism (SNP), or the like) of a biological sample that is to be, or has been, detected, measured, or quantified in a biological assay, test, process, or experiment (e.g., by PCR or sequencing assay, test, process, or experiment).

As used herein, the term "reagent" means a material or substance (e.g., in solid and/or liquid form) containing chemicals or compounds to be used in combination with a biological sample in order to facilitate a biological assay, test, process, or experiment. A reagent may comprise a combination of at least one nucleotide, at least one oligonucleotides, at least one primer, at least one polymerase, at least one salt, at least one buffering agent, at least one dye (e.g., control dye and/or binding dye), at least one marker, at least one probe, at least one enhancing agent, at least one enzyme, at least one detergent, and/or at least one blocking agent. The reagent may comprise at least one master mix (MMx) containing, for example, a combination of at least one polymerase, at least one nucleotide, at least one salt, at least one buffering agent, at least one dye (e.g., control dye and/or binding dye), and/or at least one enhancing agent. In some cases, the MMx may include one or more DNA binding dyes (e.g., a SYBR dye) or other chemicals.

As used herein, the term "reaction solution", "reaction mix", and "reaction build" means a solution or mixture containing both a biological sample and one or more reagents. The reaction solution, reaction mix, or reaction build may be used in conjunction with one or more of PCR (e.g., qPCR, dPCR, multiplex dPCR), fetal diagnostics, viral detection, quantification standards, genotyping, sequencing, sequencing validation, mutation detection, detection of genetically modified organisms, rare allele detection, and/or copy number variation, or the like.

As used herein, phrases such as "A is specific to B" means A is configured to chemically react or combine with B, or some portion of B, so as to produce a predetermined, detectable effect that may be used to determine the presence or the amount of B or some portion of B.

According to embodiments of the present invention, a reaction solution may be segregated, distributed, or divided between a plurality of relatively small reaction sites, reaction volumes, or reaction chambers to form a plurality of distributed reaction solutions. The reaction sites for embodiments of the present invention are generally disclosed herein as being contained in wells or through-holes located in a substrate material. However, other forms of reaction volumes or reaction sites according to embodiments of the present invention may be used including, but not limited to, reaction volumes located within indentations on a substrate, spots of a reaction solution distributed on the surface a substrate, test sites or volumes of a microfluidic system, or small beads or spheres containing respective portions of a reaction solution.

While devices, instruments, systems, and methods according to embodiments of the present invention are generally directed to dPCR and qPCR, alternative embodiments not relating to the present invention may be applicable to any amplification or PCR processes, experiments, assays, or protocols where a large number of reaction sites are processed, observed, and/or measured. In a dPCR assay or experiment according to embodiments of the present invention, a dilute reaction solution containing a relatively small number of at least one target is segregated, distributed, or divided into a large number of very small test samples or volumes, such that the vast majority at least some of these samples or volumes contains either one molecule of the target nucleotide sequence or none of the target nucleotide sequence. When the samples are subsequently thermally cycled in a PCR protocol, procedure, assay, process, or experiment, the individual samples containing the one or more molecules of the target nucleotide sequence are greatly amplified and produce a positive, detectable detection signal, while the individual samples containing none of the target(s) nucleotide sequence are not amplified and do not produce a detection signal, or a produce a signal that is below a predetermined threshold or noise level. Using Poisson statistics, the number of target nucleotide sequences in the original solution may be correlated to the number of samples producing a positive detection signal. In some embodiments, the detected signal may be used to determine a number, or number range, of target molecules contained in an individual sample or volume. For example, a detection system may be configured to distinguish between samples containing one target molecule and samples containing two or at least two target molecules. Additionally or alternatively, the detection system may be configured to distinguish between samples containing a number of target molecules that is at or below a predetermined amount and samples containing more than the predetermined amount. In certain embodiments, both qPCR and dPCR processes, assays, or protocols are conducted using a single the same devices, instruments, or systems, and methods.

Methods and systems described herein may be implemented in various types of systems, instruments, and machines such as biological analysis systems. For example, various embodiments may be implemented in an instrument, system or machine that performs polymerase chain reactions (PCR) on a plurality of samples. While generally applicable to quantitative polymerase chain reactions (qPCR) where a large number of samples are being processed, it should be recognized that any suitable amplification or PCR method may be used in accordance with various embodiments described herein. Suitable methods include, but are not limited to, digital PCR (dPCR), allele-specific PCR, asymmetric PCR, ligation-mediated PCR, multiplex PCR, nested PCR, qPCR, genome walking, and bridge PCR, for example. Furthermore, as used herein, thermal cycling may include using a thermal cycler, isothermal amplification, thermal convection, infrared mediated thermal cycling, or helicase dependent amplification.

PCR is an amplification process that relies on thermal cycling, which typically use repeated cycles of a process for nucleic acid melting and enzymatic replication of nucleic acids. PCR methods typically use repeated thermal cycling involving alternately heating and cooling the PCR sample to provide a series of temperature steps for a repeated number of cycles. These thermal cycling steps may be used first to physically separate nucleic acids, such as separating the two strands in a DNA double helix, at a high temperature in a process called melting. At a subsequent lower temperature, the strands are then used as the template in synthesis by a polymerase to selectively amplify one or more target molecules during an annealing phase and an extension phase. Example polymerases include heat-stable polymerase such as, for example, Taq (Thermus aquaticus) polymerase. The selectivity of PCR results from the use of primers (short nucleic acid fragments) that are complementary to the target molecules for amplification under specific thermal cycling conditions. The primers may be used in combination with a polymerase to enable selective and repeated amplification of the target molecules.

In qPCR, amplification of a target molecule may be detected in real-time during the amplification process (e.g., at different cycles during a thermal cycling process) such that the amount of initial and/or amplified target molecule may be quantified. An indicator of amplification, such as fluorescence, may be used in connection with qPCR. Dyes, such as a SYBR^{®} dye or Taqman^{®} florigenic probes, may be leveraged during PCR and amplification of the target molecule may be detected based on the fluorescence exhibited from a reaction site or reaction volume.

In dPCR, a sample to be analyzed may be segregated, distributed, divided, or partitioned so that target molecules within a reaction solution are localized within only some of a plurality of separate reaction sites. The reaction solution may be segregated, distributed, divided, or partitioned by a dilution process such that the reaction solution portion contained in each separate reaction site includes only one copy of the target molecule, approximately one copy of target molecule, or no copies of the target molecule. As in qPCR, dPCR may progress by exposing the segregated, distributed, divided, or partitioned reaction solution to an amplification assay designed to amplify the target molecules. For example, thermal cycling may be performed such that a target or template nucleic acid sequence is amplified within reaction sites that include the target or template. Reaction sites in which amplification takes place may exhibit indicators of the amplification, and such indicators may be detected. Based on the number of reaction sites producing amplification, a concentration of nucleic acids in that reaction site may be determined.

In an embodiment, an ion based detection system may be used to identify and/or quantify an initial or final amount of a target nucleic acid or molecule. During nucleic acid amplification, an H+ ion may be released. For example, during an extension stage of nucleic acid replication, as the backbone (e.g., deoxyribosephosphate backbone) is lengthened, hydrogen ion, H+, from the hydroxide group on the complimentary strand may be released. Thus, in accordance with various exemplary embodiments of the present disclosure, an exemplary indicator of amplification may include a magnitude or change in pH, charge, surface potential, current, or voltage. Based on the number or concentration of the indicators, an amplification and/or amount of the target nucleic acid or molecule may be quantified.

Conventional PCR techniques rely on relatively complex systems in order to perform thermal cycling and detection, including one or more of thermal cycling blocks, temperature control heaters, and delicate electronics and optical equipment to detect and obtain fluorescence data. Because of this, conventional systems pose issues in attempting to deliver adequate PON support (sometimes referred to as point-of-care (POC)) solutions). Various exemplary embodiments therefore contemplate PCR analytical assemblies that can include integrated and/or relatively easily coupled detection and/or thermal cycling components that provide the ability to have a relatively light-weight, easy-to-use device for achieving a PON system. In various exemplary embodiments, sensors used to detect or quantify indicators of amplification of a target molecule can rely on circuitry that is readily embedded and integrated into a dielectric, or similar-type, substrate of a reaction device, rather than, for example, relying on complex delicate optics-based detection schemes utilized in prior art systems.

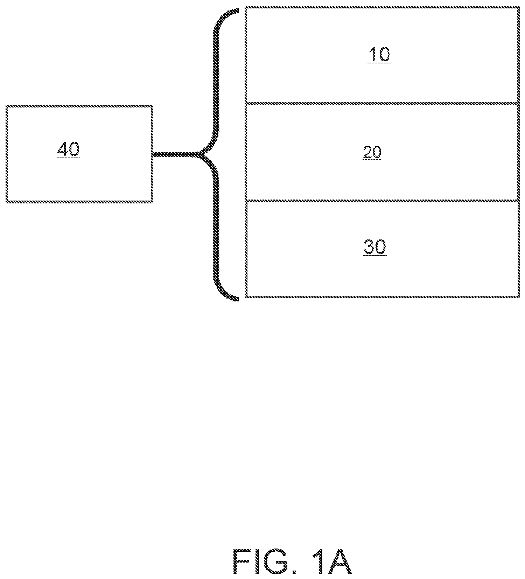

Referring to FIG. 1A, in certain embodiments, a system 10 for detecting amplification of one or more target molecules of a reaction solution comprises reaction device 10, a temperature controller 20 configured to control a temperature of the reaction device 10 and/or the reaction solution received by the reaction device 20, and detector 30 configured to detect or measure an amount of the one or more target molecules contained in the reaction solution. The system 10 may also comprise an electronic system 40 comprising one or more of a computer system, and personal computer, an electronic tablet device, smart phone, or an integrated microprocessor integrated into a common housing with one or more of the reaction device 10, the temperature controller 20, or the detector 30. The electronic system 40 comprises an electronic processor, one or more input or output devices (e.g., an input and/or output port, a keyboard, monitor, mouse controller, external storage, or the like), and an electronic memory configured to store data and instructions for controlling and/or receiving information from the reaction device 10, the temperature controller 20, or the detector 30.

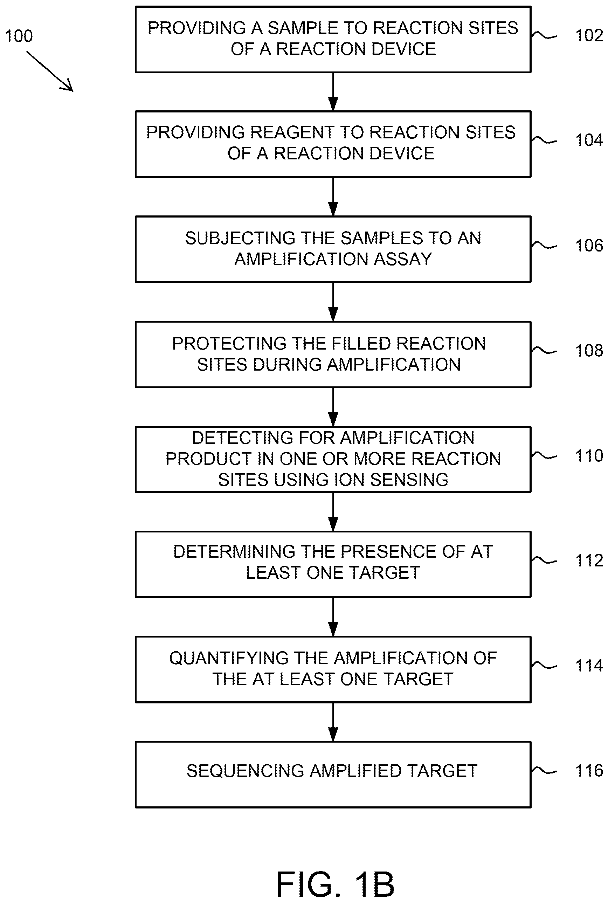

Referring to FIG. 1B, in certain embodiments, a method 100 comprises a method for detecting the presence of one or more target molecules in accordance with at least one embodiment of the present disclosure. The method 100 may be performed with a sample chip-based assembly, a circuit board comprising reaction wells and/or through holes, or with any other suitable reaction device and/or detection scheme for an amplification procedure. The elements of method 100 illustrated and described herein are exemplary, and one or more elements may be omitted or moved relative to the other elements. Method 100 may also include other elements not shown here. Also, in certain embodiments, one or more of to the elements shown in FIG. 1B may be omitted.

In general, in method 100 or another similar method according to embodiments of the present disclosure, a plurality of reaction sites, reaction volumes, or reaction chambers may be loaded with a reaction solution designed to amplify a target molecule. One or more reaction sites may be loaded with a reaction solution configured to amplify one or more target molecules that may be specific to a particular polynucleotide sequence, DNA or RNA molecule, organism, and/or virus, or the like. A sample solution, reagent, or reaction solution may be flowed over and received by at least some of the reaction sites. Once the reaction device is loaded, the reaction solution may be subjected to an amplification process or assay, for example, by thermal cycling or by isothermal amplification at predetermined temperature. In an example, at least one reaction site contains at least one target molecule that is amplified during an amplification process or assay. The amplification of a target molecule may indicate the presence, the initial quantity, and/or the final quantity of a target molecule in the distributed reaction solution that may be associated with particular polynucleotide sequence, DNA or RNA molecule, organism, and/or virus, or the like. The details of method 100 are further described below.

At element 102 of method 100, a biological sample is segregated, distributed, or divided among a plurality of reaction sites located within a reaction device, fluidic device, sample holder, or other such device. For example, a sample solution or reaction solution may be provided to the plurality of reaction sites of a reaction device that support an amplification reaction of a target molecule specific to an organism or virus. The reaction device may comprise, for example, a fluidic device, microfluidic device, titer device, microtiter plate, a spotted or through-hole array, a plurality of beads or spheres containing respective portions of a reaction solution, or any other suitable reaction device.

At element 104 of method 100, a reagent is segregated, distributed, or divided among the plurality of reaction sites located within a reaction device after element 102 has been completed. In certain embodiments, elements 102 and 104 of method 100 are switched in order, so a reagent is first segregated, distributed, or divided among the plurality of reaction sites, followed by segregating, distributing, or dividing a biological sample among the plurality of reaction sites. In other embodiments, elements 102 and 104 are combined. For example, a reagent and a sample may be mixed together to form a reaction solution, wherein the mixed reaction solution is then segregated, distributed, or divided among the plurality of reaction sites.

The reagents may, when combined with a target molecule, may be configured to provide amplification during a PCR assay or process. In an exemplary embodiment, reagents may be selected that facilitate PCR amplification in water or low ionic strength buffer. In some instances, a low buffering solution or low buffering reagents will facilitate the detection of H+ ion released during PCR amplification. The reagents may comprise components of an amplification assay, such as a Taqman^{™} assay. Exemplary embodiments in which a dye is utilized may include an intercalating dye, electrochemical dye, or any other suitable dye. In an embodiment, the reaction sites may contain reagents in lypholized form. One exemplary technique for loading reagents into reaction sites, particularly for analytical assemblies that contain an array of reaction sites of less than a microliter, includes spotting the reagents into the reaction sites via ink jet printing technology. Other available techniques to spot the reagent into reaction sites are UV light or photolithography based patterning to immobilize primers inside the reaction sites.

In an embodiment, a reaction site or reaction chamber may contain reagents that are specific to polynucleotide sequence, DNA or RNA molecule, organism, and/or virus. For example, a reaction site may contain one or more reagents that are configured for use in an amplification assay for a specific target molecule. In an exemplary embodiment, one or more primers of the amplification assay may be selected such that the assay causes amplification of a specific target molecule when the reaction site is subject to thermal cycling or isothermal amplification, as described herein.

For example, the reagents may be specific to a nucleic acid that codes for a particular type of protein in an organism, such as a bacterium, or a particular type of virus. Accordingly, when the reaction site contains the target molecule that is specific to the particular type of bacterium or virus, and is subsequently subjected to thermal cycling, the reaction site will undergo PCR amplification of the target molecule. In an embodiment, primers may be designed to target particular nucleic acids from certain organisms, such as bacteria, or certain viruses. For example, a primer pair may be designed to target molecules particular to one or more strains of human immunodeficiency virus (HIV), one or more strains of tuberculosis (TB), one or more strains of human papillomavirus (HPV), and the like. In an embodiment, a primer pair may be designed to target a nucleic acid from a particular strain of HIV, and a plurality of additional primer pairs may be designed to target molecules from variants of HIV.

In an exemplary embodiment, at least two reaction sites of the reaction device contain reaction sites to support amplification of a target molecule specific to at least two different organisms and/or viruses. For example, a first reaction site may contain reagents specific to a target molecule that codes for a first bacterium or first virus and a second reaction site may contain reagents specific to a target molecule that codes for a second bacterium or second virus.

In various exemplary embodiments, the plurality of reaction sites may contain reagents to support amplification of target molecules specific to a plurality of different organisms and/or viruses. For instance, a reaction site of the reaction device may be loaded with reagents designed to support amplification of a target molecule specific to one particular organism and/or virus, where the aggregate of the reaction sites of the reaction device contain reagents specific to hundreds, thousands, or millions of different organisms and/or viruses. In an embodiment, different reaction sites may contain reagents designed to amplify different target molecules specific to different variants of a similar organism (e.g., different strains of a bacterium or virus). Accordingly, a single chip may be loaded with different reagents (e.g., primer configurations), such that multiple target molecules may be amplified on the same chip.

In an exemplary embodiment, a plurality of adjacent reaction sites may be utilized as a single subset, or a single group of reaction sites, where the plurality of reaction sites may contain reagents specific to the same organism or virus, or in some embodiments, reagents designed to amplify the same target molecule(s). For example, the plurality of adjacent reaction sites may include an N x M polygon of reaction sites, a circle comprising a determined diameter of reaction sites, or may be adjacent to one another in any other suitable manner. The plurality of adjacent reaction sites may be loaded with reagents specific to the same organism and/or virus such that amplification hosted by the group of reaction sites may indicate the presence of the organism and/or virus in a sample or reaction solution, as described below.

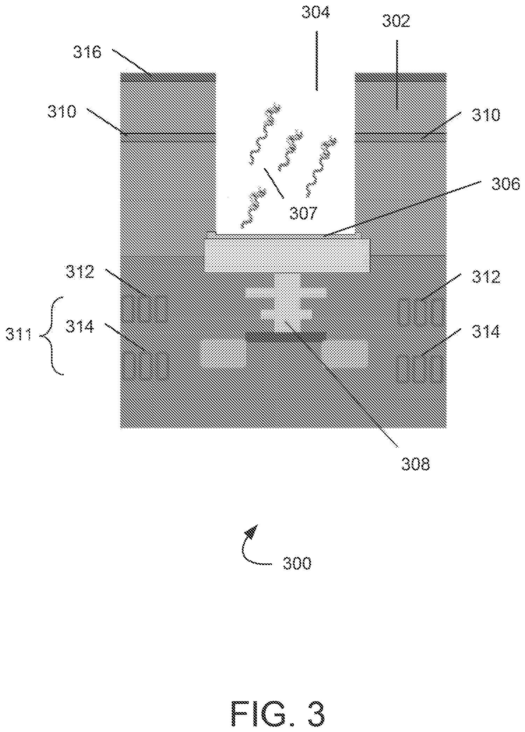

In one exemplary embodiment, a reaction device may be a chip or a chip-based device, for example, as illustrated in FIGS. 3-6 and 7A-7C, described in further detail below. Accordingly, the plurality of reagents may be segregated among the plurality of reaction sites of chip 400. In another embodiment, the reaction device may be a chip or circuit board that includes a plurality of through holes, and the plurality of reagents can be segregated among the plurality of through holes. Reaction sites can have a plurality of configurations such as for example, microwells or through holes, and may be a variety of different shapes (e.g. square, hexagonal, round, square bottom, and the like).

In one embodiment, the reaction sites may be through holes where both ends of the sites are open. For example, the bottom of the reaction sites may be fabricated such that the surrounding area of the sensing element is completely or partly etched out in order to connect the reaction sites to open space between the bottom and other supporting elements. A sensing element (such as a field effect transistor, as described herein) may thus be disposed inside of the reaction site or at the height of the lower edge of the reaction sites. The reaction reagent may be loaded into the through hole and confined within the through hole by the action of surface tension. The top and bottom gap between the reaction sites and sealing elements may then be filled with inert liquid or fluid to insulate the reaction mixture during thermal cycling or isothermal amplification.

In an embodiment, the plurality of reaction sites and plurality of reagents may be suitable for performing one or more of PCR, qPCR, or dPCR. For example, reaction sites may have a volume ranging from 1nL (nanoliter) to 100µL(micro liter), and may be used to perform qPCR assay or test, and/or the reaction sites may have a volume ranging from 1pL(picoliter) to1µL, and may be used to perform dPCR assay or test. Exemplary reaction well sizes (e.g., diameter) include 1 micron for dPCR configured reaction sites and 100 microns for qPCR configured reaction sites. In an embodiment, at least a first group of the reaction sites may be configured for performing qPCR and at least a second group of the reaction sites may be configured for performing dPCR.

In an embodiment, the sample may include a composite of nucleic acids derived from a host. For example, the sample may be obtained from a procedure performed on a human patient, where the sample is extracted from blood, sputum, a buccal swab, and any other suitable source from the human patient. Accordingly, the sample will contain nucleic acids specific to a plurality of different organisms and/or viruses present in the human patient. The sample may include, in an exemplary embodiment, purified and isolated DNA and/or RNA. In other embodiments, the DNA and/or RNA may not be purified.

In an embodiment, the sample can be loaded into the reaction sites by centrifugation or pressure flow. For example, the sample may be pipetted onto the reaction device (e.g., electronic chip), and loaded into the plurality of reaction sites by centrifugation, pressure (e.g., air flow), or any other suitable loading technique known in the art.

In an embodiment, a reaction solution may include a sample and a plurality of reagents for amplification of one or more target molecules. For example, the reaction solution may be prepared by mixing one or more target molecules with one or more corresponding reagent and subsequently segregated, distributed, or divided among a plurality of reaction sites of a reaction device. In certain embodiments, a plurality of reaction sites may be pre-loaded with one or more target molecules that are segregated, distributed, or divided among the reaction sites and then subsequently loaded with one or more reagents for amplification of the one or more target molecules. In other embodiments, a plurality of reaction sites may be pre-loaded with one or more reagents for amplification that are segregated, distributed, or divided among the reaction sites and then subsequently loaded with one or more target molecules.

At element 106 in FIG. 1B, the segregated reaction sites may be subjected to an amplification assay. For example, the reagents in the reaction sites and the sample portions may be subjected to an amplification assay, whereby amplified product based on the contents of the reaction site and the amplification assay is produced in at least one reaction site or reaction chamber.

The plurality of reaction sites of the reaction device may contain reagents to support amplification of a plurality of polynucleotide sequence, DNA or RNA molecule, organism, and/or virus, where at least some of the reaction sites may contain reagents to support amplification of a target molecule specific to one organism and/or virus. In such an embodiment, mapping of the reaction device may occur so as to associate a particular organism and/or virus with the reaction sites that contain reagents specific to the particular organism and/or virus. For example, a map may be generated when loading the reaction device with reagents specific to organisms such that the map may indicate associations between the existence of nucleic acids for particular organisms and/or viruses and one or more reaction sites (or groups of reaction sites) based on detecting an indicator of amplification. In another example, the reaction sites of the reaction device may be loaded based on the associations indicated in a map (e.g., pregenerated map).

In an embodiment, column and/or row designations may be used to accomplish the mapping. For example, reagents specific to a particular target molecule may be loaded into one or more reaction sites, and these reaction sites may be identified by their relative column number(s) and row number(s) (e.g., relative to the array of reaction sites on the reaction device). Accordingly, reagents specific to a particular nucleic acid may be associated with reaction sites in predetermined columns and rows. Based on the thermal cycling and PCR techniques described herein, detected amplification in these predetermined columns and rows indicates the presence of the particular target molecule within the sample. In some embodiments, the mapping may be specific to reaction sites configured to perform qPCR, and reaction sites configured to perform dPCR may contain reagents specific to the same target molecule.

In an exemplary embodiment, the reaction sites are subjected to an amplification assay (e.g., including thermal cycling) such that nucleic acid amplification occurs when the reagents contained in the reaction sites or reaction chamber are specific to one or more target molecules present in the sample portion at or in a given reaction site. In other words, when the reagents contained in a reaction site are specific to a target molecule of an organism and/or virus, and the specific target molecule of the organism and/or virus is present in the reaction site, the target molecule will be amplified. In an exemplary embodiment, a plurality of reaction sites may host nucleic acid amplification, where the plurality of reaction sites contain reagents specific to amplify a target molecule in the reaction site.

Referring again to FIG. 1B, at element 108 of the depicted workflow, the segregated reaction sites may be protected during amplification. For example, the plurality of reaction sites may be subjected to thermal cycling during an amplification process. To protect the reaction sites during this process, the reaction sites may be covered by a protective layer that is deposited either prior to or during this process. The protective layer may be hydrophobic mineral oil, or other fluid immiscible with the reaction fluid, that lies above the filled reaction sites to protect the reaction sites from contamination and/or evaporation reaction solution, sample, and/or target molecule(s). In an exemplary embodiment, the immiscible fluid may be flushed over the reaction device. For example, after the sample or reaction solution loaded in the wells, any excess fluid thereof can be removed by pipette. The sample or reaction solution loaded into the reaction sites will remain due to the surface tension within the reaction sites. Mineral oil may then be dispensed to isolate the reaction sites. In an embodiment, the reaction device may include a flow cell or reservoir with inlet and outlet openings to push or flow oil in and air out, thus providing a seal.

At element 110 of FIG. 1B, amplified product may be detected in one or more reaction sites. For example, one or more of the reaction sites may host nucleic acid amplification based on the reagents contained in the reaction site, the sample portion, and the amplification assay.

In an exemplary embodiment, amplified product of at least one target molecule may be indicated based on the use of an ion detection technique or similar electronic-based technique. For example, amplification can be detected by sensing a change in charge or voltage using field effect transistors associated with the corresponding plurality of reaction sites. As above, the nucleic acid amplification reaction may release a hydrogen ion, H+, from the hydroxide group on the complimentary strand. An exemplary indicator of amplification presented by the reaction sites may thus be a pH level change based on released hydrogen ions. Corresponding field effect transistors associated with the plurality of reaction sites can detect a change in surface charges or voltage due to the released H+ ions. In an exemplary embodiment, the field effect transistor may be an ion-sensitive field effect transistor (ISFET) with an ion sensing layer or plate that detects the pH change as a change in current or voltage. A change in voltage built up across the sensing layer may be output from the reaction sites and/or reaction device.

In an exemplary embodiment, the sensor may include a reference electrode associated within one or more of the plurality of reaction sites. To account for a protective layer (e.g., oil or other non-conducting substance) that may overlay the reaction volumes in the reaction sites, the reference electrodes can be placed relative to a chamber of the reaction site so as to be in contact with the reaction site. For example, a reference electrode may be a layer of material disposed on or within walls comprising the reaction sites of the sensor device. The material may be a metal (e.g. Pt, TiN, Ti, Au, Ag, and the like). The thickness of the material may be from about 100nm to about 1um and may be embedded in a wall of each reaction site. For example, the reference electrode may be embedded in a wall of a reaction site at approximately half the height of each reaction site so as to ensure contact with the reaction volume. It is contemplated that the reference electrode may be fabricated within the wall of the reaction sites in accordance with known fabrication techniques. The embedded reference electrode may be used as a voltage reference for corresponding ISFETs to sense the change in charge/voltage exhibited by a reaction volume.

With reference again to FIG. 1B, at element 112, a nucleic acid sequence of at least one organism or virus may be determined to be present in the sample. A reaction site that hosts nucleic acid amplification contains reagents specific to an organism and/or virus, wherein the sample includes nucleic acid molecules specific to the same organism or virus. This coordination allows for the amplification of the target molecule present in a reaction site.

In an embodiment, based on at least one reaction site hosting nucleic acid amplification, it may be determined that a nucleic acid molecule specific to the at least one organism and/or virus is present in the sample. Accordingly, it may be determined that an organism or virus is present in the sample when a reaction site that contains reagents specific to that organism or virus hosts nucleic acid amplification. Thus, it may be further determined that the source (e.g., blood, sputum, skin, and the like from a human or animal) from which the sample was derived includes the organism or virus.

At element 114, the nucleic acid amplification occurring in one or more reaction sites may be quantified. For example, one or more of a qPCR and/or dPCR assay or process may be leveraged to amplify one or more target molecules, and the amplification may then be quantified based on quantification techniques associated with each or either of the dPCR and qPCR amplification techniques. Quantifying the amplification of the target molecule is optional, and some embodiments may perform the method of FIG. 1B without such quantifying.

In an embodiment leveraging a qPCR assay or process, an indicator of amplification (e.g., fluorescence and/or pH, charge, current or voltage) may be sensed from a given reaction site hosting nucleic acid amplification during thermal cycling, for example, at a predetermined stage during a round of thermal cycling. In an embodiment leveraging a dPCR assay or process based on the detected indicator and a known (e.g., approximately known) initial quantity of target molecule in each reaction site, a quantity for the target molecule post-amplification may be determined. Various exemplary embodiments may leverage both qPCR and dPCR. In an embodiment implementing both qPCR and dPCR on the same chip, some population of reaction sites may support qPCR techniques for genomic detection (e.g., detection of genomic variants) and another population of reaction sites may support dPCR techniques for quantitation of the target molecule(s).

At element 116 of FIG. 1B, the amplified target molecule may be sequenced. In an embodiment, the reaction sites that host nucleic acid amplification when performing a method according to FIG. 1B may contain a plurality of copies of a particular target molecule produced as a product of the amplification. In an embodiment, sequencing, such as next generation sequencing (NGS), may be performed such that the amplified target molecule may be used as subsequent template nucleic acid strands for a sequencing assay or process. For example, the reaction device that includes the plurality reaction sites may further be designed to implement sequencing reactions, such as for example using ion sensing technology to verify incorporation events as those having ordinary skill in the art are familiar with.

In an exemplary embodiment, sequencing, such as sequencing-by-synthesis techniques, may be performed on the reaction sites that contain amplified product to sequence the copies of target molecule molecules present in a chamber of a particular reaction site. In some embodiments, the copies of the target molecule, sometimes referred to as template nucleic acid strands, may be coupled to or associated with a support, such as a microparticle, bead, or the like. In other exemplary embodiments, the template nucleic acid may be associated with a substrate surface or present in a liquid phase (e.g., in solution in the reaction volume) with or without being coupled to a support.

During a typical sequencing reaction, a primer may be annealed to a template nucleic acid strand to form a primer-template duplex, and a polymerase may be operably bound to the primer-template duplex so that it is capable of incorporating a nucleotide onto a 3' end of the primer. As used herein, "operably bound" may refer to the primer being annealed to a template strand so that the primer's 3' end may be extended by a polymerase and that a polymerase is bound to the primer-template duplex, or in close proximity thereof, so that extension can take place when dNTPs are flowed. The primer-template-polymerase complex may be subjected to repeated exposures of different nucleotides. If a nucleotide(s) is incorporated, then the signal resulting from the incorporation reaction is detected. A wash step may be performed to remove unincorporated nucleotides prior to the next nucleotide exposure. After repeated cycles of nucleotide addition, primer extension, and signal acquisition, the nucleotide sequence of the template strands may be determined.

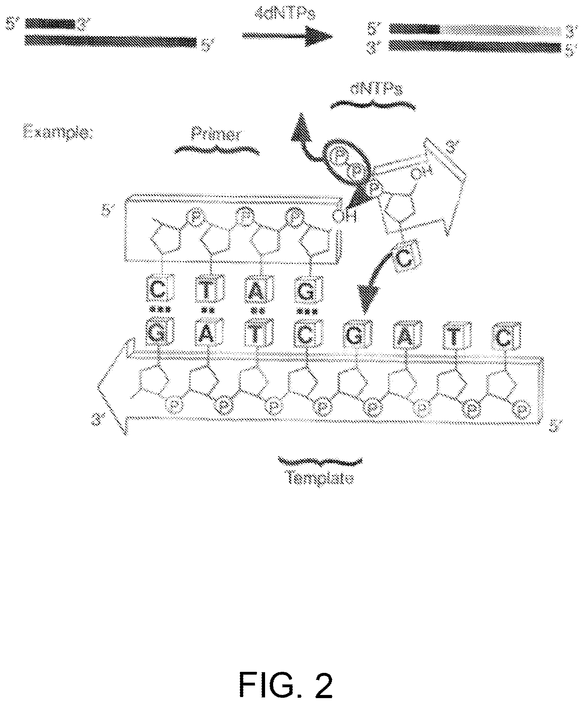

In some embodiments, the incorporation reaction based on a flowed dNTP generates or results in a product or constituent with a property capable of being monitored and used to detect the incorporation event. For example, FIG. 2 illustrates the extension of a complementary strand of a nucleic acid. As the backbone (e.g., deoxyribosephosphate backbone) is lengthened, a pyrophosphate molecule may be liberated to drive the reaction forward, as described herein in an embodiment. In an embodiment, the reaction may release a single hydrogen ion, H+, from the hydroxide group on the complimentary strand.

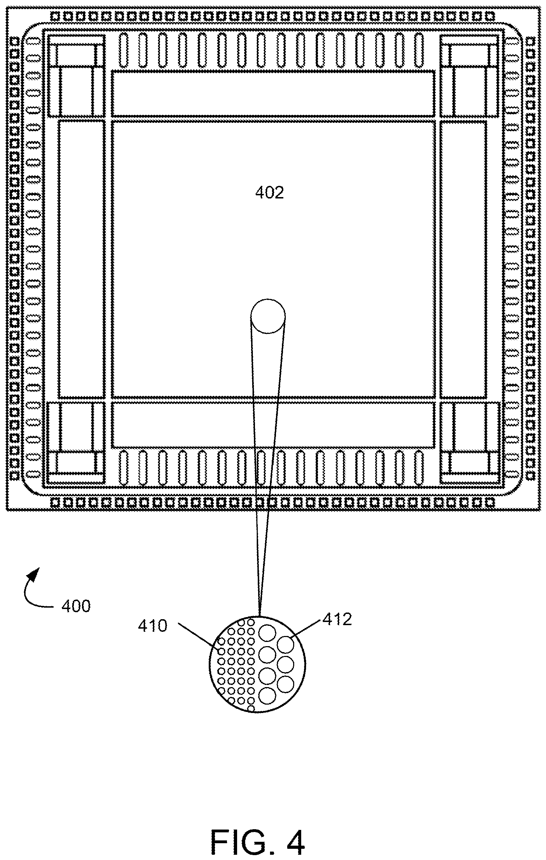

In one exemplary embodiment, the reaction device may be a Complementary Metal-Oxide Semiconductor (CMOS) chip based device, as illustrated in FIG. 4 and described in further detail below. Accordingly, the plurality of reagents may be segregated among the plurality of reaction sites of chip 400. In various exemplary embodiments, the reaction device that is used to perform a method according to FIG. 1B may be further utilized for sequencing reactions. For example, the reaction device may be configured for PCR amplification, including qPCR and dPCR amplification, and may be further configured to implement sequencing reactions based on flowed dNTPs.

Additional details of pH-based sequence detection systems and methods may be found in commonly-assigned U.S. Patent Application Publication No. 2009/0127589 and No. 2009/0026082, which are incorporated by reference in their entirety as if fully set forth herein.

In various exemplary embodiments, the reaction device implemented may enable both PCR amplification reactions and sequencing reactions within the reaction sites of the device. For example, the reaction sites may be subjected to thermal cycling and optionally subsequently subjected to dNTP reagent flows, where the reaction sites may include components to enable signal detection based on the reactions occurring in the chamber of at least some of the reaction sites.

FIG. 3 is a diagrammatic plan view of a reaction device 300 configured to permit detection of a molecule of a target molecule in accordance with various exemplary embodiments of the present disclosure. The reaction device 300 may comprise a substrate 302, one or more reaction sites 304, an ion sensitive layer 306, and sensor 308. As shown in FIG. 3, the substrate 302 may include the one or more reaction sites 304, which reaction sites 304 may be configured to contain and/or confine a sample or reaction solution 307. Substrate 302 may comprise a dielectric layer, and/or any other suitable materials. The ion sensitive layer 306 may be in contact and/or in chemical communication with the reaction site 304 and/or the reaction solution 307. Another surface of the sensitive layer 306 may be in contact and/or in electrical communication with the sensor 308. In some embodiments, the reaction site 304, or reaction sites 304 in embodiments where there is more than one reaction site, is in chemical communication with a single sensor 308. Alternatively, a given reaction site 304 may be in chemical communication with its own individual sensor, as shown in FIG. 3. In some embodiments, sensor 308 may be a chemical field-effect transistor (chemFET) sensor, an ion-sensitive field effect transistor (ISFET), or some other suitable biosensor. In some embodiments, the sensor detects, or may be used to detect, changes to ion concentration, pH, heat, enzyme activity, or any other form of energy emitted in response to a reaction occurring in the reaction site 304.

Exemplary embodiments of arrays of ISFETs used for monitoring chemical reactions, such as DNA sequencing reactions, based on the detection of ions present, generated, or used during the reactions are described in, U.S. Patent No. 7,948,015 to Rothberg et al., U.S. Patent Pub. No. 2014/0264470 to Fife et al., U.S. Patent Pub. No. 2014/0264471 to Fife et al., and U.S. Patent Pub. No. 2014/0264472 to Fife et al., which are incorporated by reference herein in their entirety as if fully set forth herein. More generally, large arrays of chemFETs or other types of chemical sensors may be employed to detect and measure static and/or dynamic amounts or concentrations of a variety of analytes (e.g. hydrogen ions, other ions, compounds, etc.) in a variety of processes. The processes may for example be biological or chemical reactions, cell or tissue cultures or monitoring neural activity, nucleic acid sequencing, etc.

In an exemplary embodiment, during use, as amplification of one or more target molecules in the sample or reaction solution 307 occurs and/or as incorporation of a nucleotide occurs in reaction solution 307 (e.g., during the extension phase), hydrogen ions are released, which may effectively alter (e.g., lower) the pH in the reaction site 304. The ion sensing layer or plate 306 may be configured to detect the pH change, for example, as a rise in charge at the sensing layer, as surface charge density changes. If enough charge builds, the surface potential (voltage) changes as the surface charge density changes, wherein the sensor 308 may read out this change in the voltage built up across the sensing layer. In some embodiments, the nucleic acid in the reaction volume may undergo an amplification reaction using any suitable method for performing such amplification reaction. Such methods may include, but are not limited to, the use of a thermal cycler or isothermal amplification, such as loop-mediated isothermal amplification (LAMP), nicking enzyme amplification reaction (NEAR), helicase-dependent amplification, recombinase polymerase amplification (RPA), or any other suitable method of performing an amplification reaction that yields a detectable reaction byproduct or target.

Reaction device 300 may further comprise an embedded reference electrode 310 that may be used to detect changes occurring in reaction site 304. The embedded reference electrode 310 may be a layer of metal deposited in a position such that it makes contact with the reaction site 304 and/or reaction solution 307. In certain embodiments, the embedded reference electrode 310 may be located approximately half way up a sidewall of a reaction site. Reference electrode 310 may be disposed within the sidewall in accordance with suitable process steps for fabricating such reference electrode. In some embodiments, sensor 308 may detect changes occurring in reaction site 304 based on the reference electrode 310. In these embodiments, the reference electrode 310 may be fabricated within or adjacent reaction site 304, and a given reference electrode may be configured relative to a given ISFET detection system communicatively linked to reaction site 304.

In some embodiments, reaction device 300 comprises a temperature controller 311 that may be configured to control the temperature of at least a portion of the reaction device 300 and/or the reaction solution 306 received by reaction site 304. For PCR or other amplification processes or assays, temperature controller 311 may comprise a thermal cycler used configured to produce thermal cycling of the reaction solution 307 during amplification. The temperature controller 311 may comprise a heating elements 312 and/or temperature sensors 314 that may be fabricated into substrate 302. Heating elements 312 and temperature sensors 314 may be located in the substrate or below the sensing layer, or in any other suitable location on the reaction device 300. In some embodiments, the heating elements 312 may be located in a cover or lid placed over an opening of the reaction site, which may serve to provide heat to the reaction site, and may or may not at least partially seal the reaction site. In some embodiments, the heating element is an infrared heat source, such as for example, a tungsten lamp or a radiation emitter. In such an embodiment, cooling can be achieved using heat dissipation or by compressed air.

In some embodiments, heating elements 312 may be addressable to perform independent thermal cycling of a reaction site or selected groups of reaction sites. Temperature sensors 314 may similarly be addressable to measure and output temperature information (e.g., of reaction volumes contained in one or more reaction sites 304). In some embodiments, heating elements 312 may leverage conductor and semiconductor materials to generate heat and/or other forms of electromagnetic radiation. In some embodiments, heating elements 312 and temperature sensors 314 may include depositions of platinum, doped polysilicon, or any other suitable material.

As an alternative to contact heating for thermal cycling, the temperature controller 311 may separate from, but in thermal contact with, the reaction device 300. Various noncontact heating methods may be employed including, but not limited to, by way of example hot-air mediated heating, utilization of IR light, laser-mediated heating induction heating, and microwave irradiation. In some embodiments, rather than being built into the reaction device, an energy source and/or sink may be coupled so as to be portable with the device. In some embodiments, the reaction device may be configured to be coupled to a Peltier or other thermal source such as a thermal block, heat pad, thin film heater or any other suitable heating source.

In various exemplary embodiments, the reaction device may be fabricated as a CMOS chip. Fabrication of one or an array of reaction sites in silicon with integrated sensors (e.g., field effect transistor sensors) for nucleic acid amplification monitoring has been described, for example, in U.S. Publication No. 2010/0301398 and Iordanov et al., Sensorised Nanoliter Reactor Chamber for DNA Multiplication, IEEE (2004) 229-232, both of which are incorporated by reference in their entirety as if fully set forth herein. Reaction sites thus fabricated might be provided with an integrated ISFET for monitoring of nucleic acid amplification. As noted by Iordanov et al. in their above-noted paper, untreated silicon and standard silicon-related materials can be inhibitors of Taq polymerase. Therefore, when silicon or a silicon-related material, e.g. silicon germanium or strained silicon is employed for fabrication of a microchip chamber or channel for nucleic acid amplification it will usually be covered with material to prevent reduction of polymerase efficiency by the silicon, such as, for example, SU8, polymethyl-methacrylate (PMMA), Perspex^{™} or glass.

In some embodiments, reaction device 300 may be loaded with a reaction solution (e.g., a biological sample and one or more reagents for amplification of one or more target molecules). In an example, one or more surface within or outside each reaction site 304 can be configured to draw in a reaction solution and maintain the reaction solution within the reaction sites. In one exemplary embodiment, a hydrophobic layer 316 may be formed or deposited on the top surface of reaction device 300, such that layer 316 is more hydrophobic than one or more interior surfaces of each reaction site 304. Thus, a sample or reaction solution can be loaded into the reaction sites by surface tension while mitigating the risk of cross-contamination. In an embodiment that implements through holes, the bottom surface or layer of a reaction device may similarly comprise a hydrophobic layer. In some embodiments, hydrophobic layer(s) 316 may comprise a film polymer, such as TEFLON^{®}, Polymide, and the like, or may comprise a chemical coating, such as silane, phosphate, phosphonate, or the like. Depositing of hydrophobic layer 316 may be achieved by spinning, dipping, and any other suitable technique.

As discussed above, in various exemplary embodiments in accordance with the present disclosure, nucleic acid amplification (*e.g.*, PCR ) may be performed using a microfabricated chip that includes an array of reaction sites into which reagents are segregated. In such a device, the reagents remain in their individual reaction sites while subjected to the amplification assay, including for example the various stages of thermal cycling.

FIG. 4 illustrates an exemplary embodiment of a reaction device comprising a sample chip 400. In an embodiment, the chip 400 may be a CMOS chip and/or may further include element array 402. In an embodiment, an element in the array 402 may include an ion-sensitive field effect transistor (ISFET) sensor, a chemical field-effect transistor (chemFET) sensor, another suitable biosensor, and/or any other transistors and electrical components. The array 402 may be arranged as a plurality of rows and columns and may be an array of reaction sites (e.g., microwells, nanowells, picowells, through holes, micro-particles, beads, and the like). For example, the reaction sites may be suitable for hosting or providing PCR (e.g., dPCR and/or qPCR) assays or reactions for target molecule amplification and detection and/or nucleic acid sequencing reactions. In an embodiment, the array 402 may comprise reaction sites having dimensions or volumes that differ from one another (e.g., comprising a first plurality of reaction sites characterized by a first dimension or volume, and a second plurality of reaction sites characterized by a first dimension or volume that is different from that of the reaction sites in the first plurality of reaction sites). For example, the size for a first set of reaction sites 410 configured for dPCR (e.g., having a size or diameter of approximately one micron), while the size or diameter of a second set of reaction sites 412 may be configured for qPCR (e.g., having a size or diameter of approximately 100 microns). In an embodiment, chip 400 may include a resistive heating element and/or Peltier device that allows for thermal cycling.

In some exemplary embodiments, a chip (e.g., silicon chip or chip 400) may be used to perform the PCR process. For example, a sample or reaction solution containing one or more target molecules may be loaded onto the chip prior to amplification as previously described. In some embodiments, the reaction solution may be amplified outside of the chip, or enriched, and then the amplified target(s) may be loaded onto the chip. Once on the chip, the reaction solution may undergo any suitable reaction to release a detectable by-product. In some embodiments, reaction volumes are formed by shearing the reaction solution into smaller reaction volumes using an immiscible fluid, which are then loaded onto the chip.

In an embodiment, a sample or reaction solution can be loaded into the reaction sites by centrifuge spinning or pressure driven flow. After loading, excess liquid can be removed by pipette. The reaction solution loaded into the individual reaction sites may remain within the sites due to the surface tension within the reaction sites. Mineral oil may then be dispensed to isolate the reaction sites and avoid cross contamination among different reaction sites during a reaction. In an embodiment, the reaction device may include a flow cell or reservoir with inlet and outlet openings to push or flow oil in and air out, thus providing a seal. The top surface (and bottom surface for through hole embodiment) as well as the interior of the reaction sites can be modified so as to draw in the reaction reagents and maintain the reaction mixture within the reaction sites. In one exemplary embodiment, the top and bottom surfaces of the chip are modified to be more hydrophobic than the interior of the reaction site to make the reaction reagent load into the reaction sites by surface tension. In another embodiment, the reagent is adjusted to preferentially remain inside the reaction site once it is loaded into the reaction site.

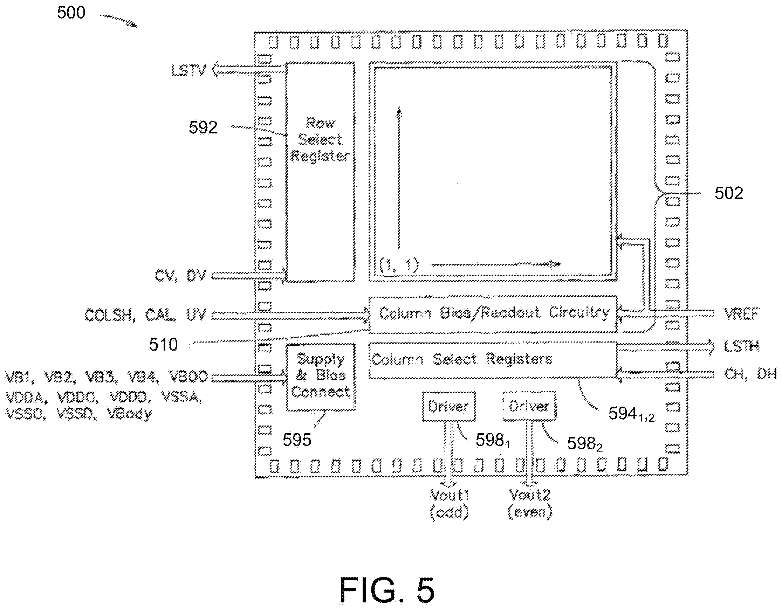

FIG.5 illustrates a block diagram of an exemplary reaction device comprising a CMOS IC chip implementation of an ISFET sensor array 500 based on the column and row designs, according to one embodiment of the present disclosure. In one aspect of this embodiment, the array 500 includes columns 502 with corresponding column bias/readout circuitry 510 (e.g., one for a column), wherein a column includes 512 geometrically square pixels (i.e., reaction sites). In another aspect, the entire array (including pixels together with associated row and column select circuitry and column bias/readout circuitry) may be fabricated on a semiconductor die as an application specific integrated circuit (ASIC) having dimensions of approximately 7 millimeters by 7 millimeters. While an array of 512 by 512 pixels is contemplated in the embodiment of FIG. 5, it should be appreciated that arrays may be implemented with different numbers of rows and columns and different pixel sizes according to other embodiments. Further, an array including reaction sites of varying sizes, as described herein, may be used to implement contemplated embodiments.

Also, it should be appreciated that arrays according to various embodiments of the present invention may be fabricated according to conventional CMOS fabrications techniques, as well as modified CMOS fabrication techniques (e.g., to facilitate realization of various functional aspects of the chemFET arrays, such as additional deposition of passivation materials, process steps to mitigate trapped charge, etc.) and other semiconductor fabrication techniques beyond those conventionally employed in CMOS fabrication. Additionally, various lithography techniques may be employed as part of an array fabrication process. For example, in one exemplary implementation, a lithography technique may be employed in which appropriately designed blocks are "stitched" together by overlapping the edges of a step and repeat lithography exposures on a wafer substrate by approximately 0.2 micrometers. In a single exposure, the maximum die size typically is approximately 21 millimeters by 21 millimeters. By selectively exposing different blocks (sides, top & bottoms, core, etc.) large chips can be defined on a wafer (up to a maximum, in the extreme, of one chip per wafer, commonly referred to as "wafer scale integration").

In one aspect of the array 500 shown in FIG. 5, the first and last two of columns 502 as well as the first two pixels and the last two pixels of the columns 502 (e.g., two rows and columns of pixels around a perimeter of the array) may be configured as "reference" or "dummy" pixels. For example, the topmost metal layer of a dummy pixel's ISFET (coupled ultimately to an ISFETs polysilicon gate) may be tied to the same metal layer of other dummy pixels and may be made accessible as a terminal of the chip, which in turn may be coupled to a reference voltage VREF. The reference voltage VREF also may be applied to the bias/readout circuitry of respective columns of the array. In some exemplary implementations, preliminary test/evaluation data may be acquired from the array based on applying the reference voltage VREF and selecting and reading out dummy pixels, and/or reading out columns based on the direct application of VREF to respective column buffers (e.g., via the CAL signal), to facilitate offset determination (e.g., pixel-to-pixel and column-to-column variances) and array calibration.

In FIG. 5, various power supply and bias voltages for array operation are provided to the array via electrical connections (e.g., pins, metal pads) and labeled for simplicity in block 595 as supply and bias connections. The array 500 of FIG. 5 also includes a row select shift register 592, two sets of column select shift registers 594_{1,2} and two output 65 drivers 598₁ and 598₂ to provide two parallel output signals from the array, Voutl and Vout2, representing sensor measurements. The various power supply and bias voltages, control signals for the row and column shift registers, and control signals for the column bias/readout circuitry shown in FIG. 5 are provided by an array controller, which also reads the output signals Voutl and Vout2 (and other optional status/diagnostic signals) from the array 500. In another aspect of the array embodiment shown in FIG. 5, configuring the array such that multiple regions (e.g., multiple columns) of the array may be read at the same time via multiple parallel array outputs (e.g., Voutl and Vout2) facilitates increased data acquisition rates. While FIG. 5 illustrates an array having two column select registers and parallel output signals Voutl and Vout2 to acquire data simultaneously from two columns at a time, it should be appreciated that, in other embodiments, arrays according to the present disclosure may be configured to have only one measurement signal output, or more than two measurement signal outputs, more dense arrays according to other embodiments may be configured to have four our more parallel measurement signal outputs and simultaneously enable different regions of the array to provide data via the four our more outputs.

Various other configurations of array 500 may be implemented with embodiments including different pixel sizes, array sizes, and component configurations. Additional embodiments of reaction devices, such as CMOS chips, that can be implemented with the present disclosure may be found in commonly-assigned U.S. Patent No. 7,948,015, which is incorporated herein by reference in its entirety as if fully set forth herein.

In an exemplary embodiment, a flow cell may be used with a reaction device 610 to implement described embodiments. The process of using the assembly of an array of sensors on a chip combined with an array of reaction sites to sequence DNA in a sample may be referred to as an "experiment." Executing an experiment involved loading reaction sites with DNA and flowing several different solutions (i.e., reagents and washes) across the reaction sites. A fluid delivery system coupled with a fluidic interface can be used to flow various solutions across the wells in a controlled laminar flow with acceptably small dead volumes and small cross contamination between sequential solutions. Flow cell designs of many configurations are possible; the system and methods presented herein are not dependent on use of a specific flow cell configuration. In certain embodiments, it may be desirable that a flow cell substantially conform to one or more the following set of objectives:
suitable interconnections with a fluidics delivery system (e. g., via appropriately-sized tubing);
appropriate (for DNA sequencing, approximately 300 µm) head space above wells;
minimization of dead volumes encountered by fluids prior to their entry to the flow chamber (i.e., the enclosed space above the microwell array);
elimination of small spaces in contact with liquid but not swept by through the flow cell (to minimize cross contamination);
uniform expansion of flow from the inlet tubing to a broad/ flat front at the entry to the flow chamber;
laminar flow characteristics such that the broad/flat front profile is maintained as it traverses across the chip from inlet side to outlet side;
easy loading of beads;
manufacturable at acceptable cost easy assembly of flow cell and attachment to the chip package.

For example, a flow cell may be implemented by attaching the flow cell to a frame and gluing the frame (or otherwise attaching it) to a reaction device (e.g., chip). Alternatively, the flow cell may be implemented by integrating the frame into the flow cell structure and attaching this unified assembly to the reaction device. Further, designs may be categorized by the way the reference electrode is integrated into the arrangement.

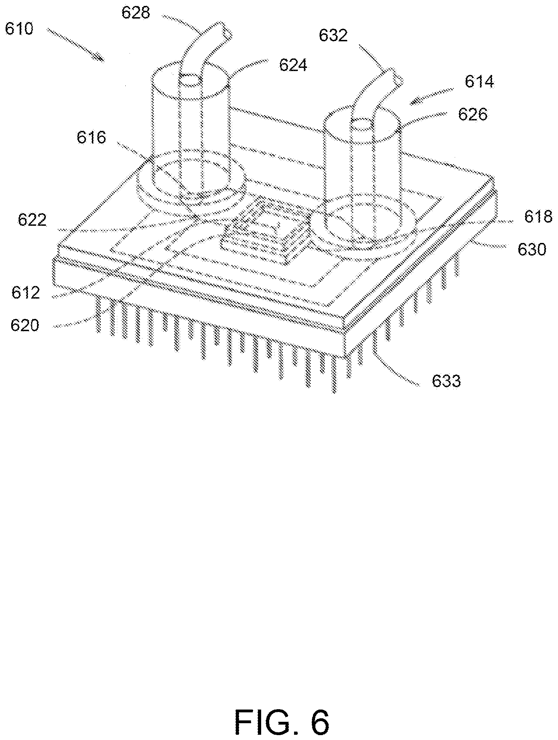

An example of a suitable experiment apparatus 600 incorporating such a flow cell is shown in FIG. 6. The apparatus 600 comprises a semiconductor chip 612 (indicated generally, though hidden) on or in which the arrays of reaction sites and sensors are formed, and a fluidics assembly 614 on top of the chip and delivering the sample to the chip for reading. The fluidics assembly includes a portion 616 for introducing fluid containing the sample, a portion 618 for allowing the fluid to be piped out, and a flow chamber portion 620 for allowing the fluid to flow from inlet to outlet and along the way interact with the material in the reaction sites. Those three portions are unified by an interface comprising a glass slide 622 (e.g., Erie Microarray Cat #C22-5128-M20 from Erie Scientific Company, Portsmouth, N.H., cut in thirds of size about 25 mmx25 mm). Mounted on the top face of the glass slide are two fittings, 624 and 626, such as nanoport fittings Part# N-333 from Upchurch Scientific of Oak Harbor, Wash. One port (e.g., 624) serves as an inlet delivering liquids from a pumping/valving system. The second port (e.g., 626) is the outlet which pipes the liquids to waste. A given port connects to a conduit 628, 632 such as flexible tubing of appropriate inner diameter. The nanoports are mounted such that the tubing can penetrate corresponding holes in the glass slide. The tube apertures may be flush with the bottom surface of the slide. On the bottom of the glass slide, flow chamber 620 may comprise various structures for promoting a substantially laminar flow across the reaction site array. For example, a series of microfluidic channels fanning out from the inlet pipe to the edge of the flow chamber may be patterned by contact lithography using positive photoresists such as SU-8 photoresist from MicroChem Corp. of Newton, Mass. The chip 612 may be mounted to a carrier 630, for packing and connection to connector pins 632.

In various embodiments, the flow cell may be used to introduce mineral oil to the reaction sites and to wash away previously deposited mineral oil. Various other configurations of the flow cell may be implemented with embodiments. Additional embodiments of flow cells that can be implemented with the present disclosure may be found in commonly-assigned U.S. Patent No. 7948015.

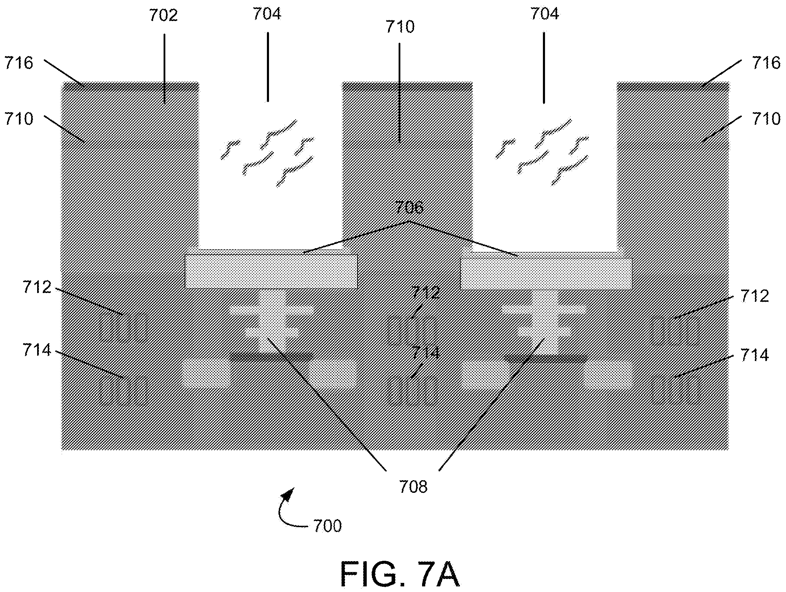

FIGS. 7A-7C are sectional views of an exemplary embodiment of a reaction device 700 that may be similar to the chip-based devices 400, 500 illustrated in FIG. 4 or FIG. 5. Reaction device 700 comprises a substrate 702 having a plurality of reaction sites 704 (only two being illustrated for simplicity). For example, reaction sites 704 may be neighboring elements of an array similar to element array 402, illustrated in FIG. 4. Substrates 702 may include dPCR reaction sites numbering in the 100,000' s or millions, approximately, and qPCR reaction sites numbering in the 100' s to 1000' s, approximately. As shown in FIG. 7A, the reaction sites 704 may be located in chemical communication with a sensing layer 706 which may be in electrical communication with at least one sensor 708. In some embodiments, a given reaction site 704 is in chemical and electrical communication with its own respective sensor 708. Embedded reference electrodes 710 may be reference electrodes used to detect changes occurring in a reaction site. In some embodiments, as illustrated, an embedded reference electrode may be included for a given reaction site. For example, the embedded reference electrode may be a layer of metal deposited in a position such that it makes contact with the reaction volume in each reaction site, as will be explained in further detail below. In an exemplary embodiment, the embedded reference electrode 710 may be located approximately half way up the wall of a reaction site, although the reference electrode may be disposed within the wall in accordance with suitable process steps for fabricating such reference electrode.

In some embodiments, sensor 708 may comprise an ion-sensitive field effect transistor (ISFET), or any other suitable biosensor. In some embodiments, sensor 708 detects changes to surface potential, ion concentration, pH, heat, enzyme activity, or any other form of energy emitted in response to a reaction occurring in the reaction site. Sensor 708 can be configured to detect the release of hydrogen ions during a reaction. For example, as the target molecule is being amplified, with a nucleotide binding occurrence (e.g., during the extension phase of replication) the system may detect a change in ion concentration and report such change as a voltage spike. In some embodiments, sensor 708 may detect changes occurring in individual reaction sites based on the individual reference electrode 710 specific to a given reaction site. In these embodiments, the reference electrode is fabricated within a reaction site, and a given reference electrode is configured relative to a given ISFET detection system communicatively linked to a reaction site.

Various exemplary embodiments in accordance with the present disclosure contemplate providing reaction devices with on-board integrated heaters to accomplish thermal cycling. In some embodiments, heating elements 712 and temperature sensors 714 may be fabricated into substrate 702. Heating elements 712 and temperature sensors 714 may be located in the substrate or below the sensing layer 706, or in any other suitable location on the device.

In some embodiments, heating elements 712 may be addressable to perform independent thermal cycling of a reaction site or selected groups of reaction sites. Temperature sensors 714 may similarly be addressable to measure and output temperature information (e.g., of reaction volumes contained in one or more reaction sites 704). In some embodiments, heating elements 712 may leverage conductor and semiconductor materials to generate heat and/or other forms of electromagnetic radiation. In some embodiments, heating elements 712 and temperature sensors 714 may include depositions of platinum, doped polysilicon, or any other suitable material.

In some embodiments, reaction device 700 may be loaded with a reaction solution. In an example, the top surface (and bottom surface for through hole embodiment) as well as the interior of the reaction sites can be modified so as to draw in the reaction reagents and maintain the reaction solution within the reaction sites. In one exemplary embodiment, hydrophobic layer 716 may be deposited on the top layer of reaction device 700 such that the top surfaces of the chip are modified to be more hydrophobic than the interior of reaction sites 704. Thus, reagents and/or a reaction solution can be loaded into the reaction sites by surface tension while mitigating the risk of cross-contamination. In an embodiment that implements through holes, the bottom layer of a reaction device may similarly comprise a hydrophobic layer. In some embodiments, hydrophobic layer 716 may comprise a film polymer, such as TEFLON^{®}, Polymide, and the like, or may comprise a chemical coating, such as silane, phosphate, phosphonate, and the like. Depositing of hydrophobic layer 716 may be achieved by spinning, dipping, and any other suitable technique.

In FIGS. 7A-7C, exemplary neighboring reaction sites in a reaction device, such as for example the chip 400 of FIG. 4 or chip 500 of FIG. 5, is used to perform at least portions of the method of FIG. 1B. For example, FIG. 7A illustrates sample reaction sites that include a reagent for performing PCR amplification of one or more target molecules, as described above. Reaction sites 704 may contain reagents designed to amplify a different target molecule.

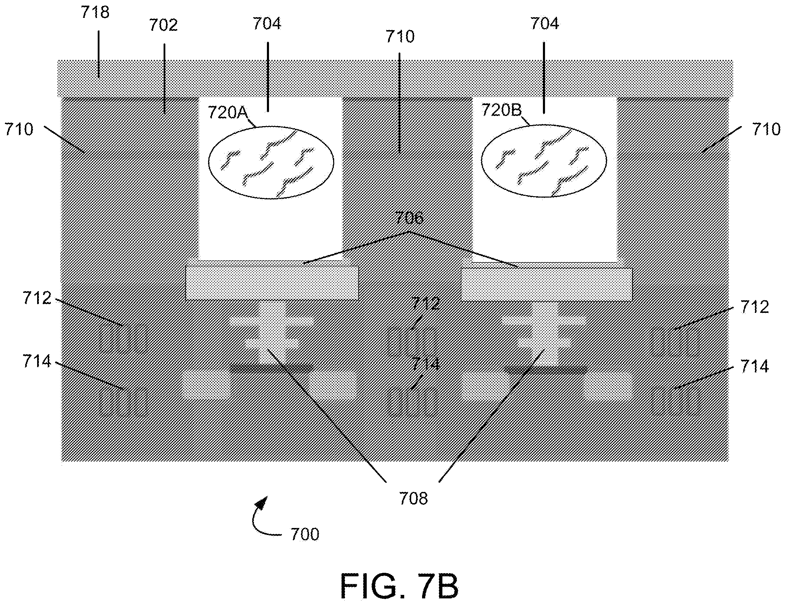

Referring to FIG. 7B, during an assay, reaction device 700 may optionally comprise a protective layer 718 deposited over the reaction sites. Protective layer 718 may be positioned over the reaction sites 704 to isolate the reaction solution in the individual each reaction site, such that contamination and/or evaporation during amplification may be avoided. In an exemplary embodiment, protective layer 718 may be an immiscible fluid layer, such as a hydrophobic mineral oil, a plastic layer, a glass layer, or any other suitable sealing layer. In some embodiments, at least a portion of the top surface of substrate 702 or hydrophobic layer 716, when included, may be coated with protective layer 718.

In certain embodiments, a dPCR assay may be performed, wherein at least some reaction sites 704 contain a reaction solution 720A that includes a target molecule, while other reaction sites 704 contain a reaction solution 720B that does not include a target molecule, for example, due to a very low concentration of the target molecule contained in the reaction solution distributed between the reaction sites 704. As discussed above, reaction device may also optionally include other reaction sites 704 that are configured for qPCR, for example, each qPCR reaction site having a volume that is much larger than the two reaction sites 704 illustrated in FIG. 7A-7C.

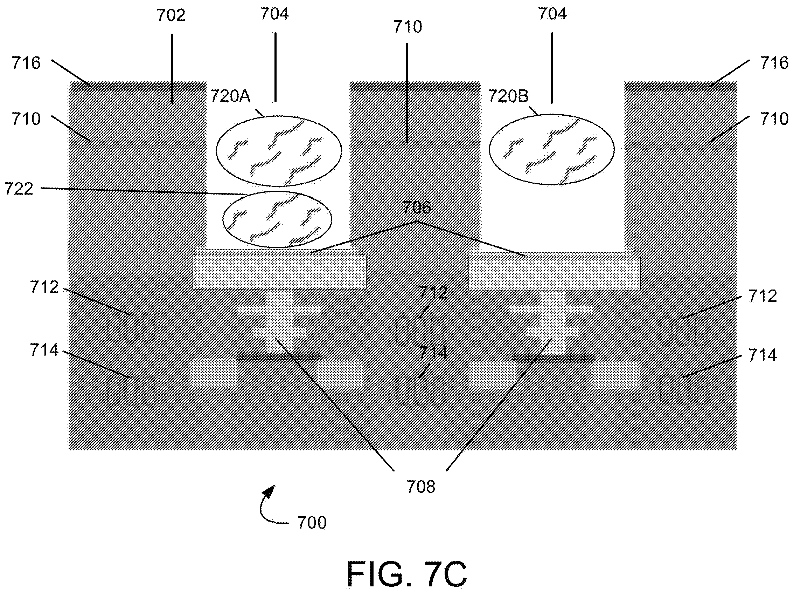

FIG. 7C illustrates the reaction device 700 post-amplification. In the illustrated example, the reaction sites 704 comprising reaction solution 720A also includes an amplified target 722 after the application assay, while the reaction sites 704 comprising reaction solution 720B does not. This is because, while both illustrated reaction sites 704 included the same reagent configured to amplify the target molecule, no target molecule was present in the reaction solution 720B. In an embodiment, protective layer 718 may be removed or washed away, as illustrated in FIG. 7C. In an embodiment, an organic solvent wash may be used to remove the mineral oil after amplification. Further reactions or analysis, such as sequencing, may be performed on reaction cites 704 containing amplified target 722 may be performed after the amplification assay.

In an exemplary embodiment, the techniques described herein may be used to detect and determine particular strands of a virus. To enable simultaneous variant detection and titer count, the reaction devices herein may be designed to accommodate both qPCR and dPCR reaction sites, as shown in FIG. 4. In some embodiments, the latter reaction sites will number in the 100,000s or millions (approximately) and may be between 100-1000 times smaller than the qPCR reaction sites, which may number in the 100s, an may contain primers for a specific variant or mutation of virus, such as human immunodeficiency virus (HIV). In some embodiments, the qPCR reaction sites may identify the particular virus variant while the dPCR reaction sites may contain more general virus assays for simultaneous virus quantitation.

For example, reaction device, such as chip 400 of FIG. 4 or chip 500 of FIG. 5, may include a first set of reaction sites configured to perform dPCR and a second set of reaction sites configured to perform qPCR. In an embodiment, the size difference between the first set of reaction sites and the second set of reaction sites may enable control of the sample or reaction solution loaded into a set. For example, a diluted sample or reaction solution may be loaded onto chip 400 and loaded into the reaction sites, and size difference between the first set of reaction sites and the second set of reaction sites may result in approximately 1 copy of a target molecule being loaded into the first set of reaction sites and a number of copies (e.g., 10,000 copies) of target molecules being loaded into the second set of reaction sites. Thus, the first set of wells may be configured to perform dPCR based on the controlled quantity of target molecule while the second set of reaction sites may be configured for qPCR.

In an embodiment, the size for the first set of reaction sites may be one micron while the size for the second set of reaction sites may be 100 microns. The dynamic range resulting from this size difference may enable the described control of loaded sample or reaction solution. In various embodiments, the first set of reaction sites may number in the 100,000s or millions, while the second set of reaction sites may number in the 10s to 100s.

In an embodiment, the reagents loaded into the first set of reaction sites may be specific to the same target molecule. For example, the first set of reaction sites may be used to perform dPCR and quantify the amount of target molecule in a sample or reaction solution. For instance, reagents (e.g., primer pairs) loaded in the first set of reaction sites may be specific to a surface protein common to a variety of strains of HIV. Accordingly, after loading of the sample or reaction solution and thermal cycling, a titer count may be determined for the various strains of the virus.

In an embodiment, the reagents loaded into the second set of reaction sites may be specific to different target molecules. For example, a number of different nucleic acids may be associated with a plurality of strains of HIV. The second set of reaction sites may be loaded with reagents (e.g., primer pairs) that are designed to amplify different target molecules associated with the plurality of strains of HIV. In an embodiment, redundant reaction sites may be leveraged, where two or more of the second set of reaction sites are loaded with reagents specific to one particular strain of HIV. Based on such a configuration, a sample or reaction solution may be loaded into the reaction sites and thermal cycling may be performed. Detected amplification in one or more of the second set of reaction sites will indicate the presence of one or more particular strain(s) of HIV associated with the reaction sites that contain amplified product. Accordingly, particular strains of HIV within a patient (e.g., from which the sample was derived) may be identified, and treatments for the patient may be specifically tailored to the identified strains. For instance, drug resistant strains of HIV may be identified, and particular treatments for these strains may be leveraged.

Accordingly, a single chip may be used to determine genotyping of the HIV virus and titer count for the virus using an efficient PCR methodology. Here, the single chip measures both the quantity of the virus and the variants (or genotypes) present in the sample.

## Claims

1. A method for detecting nucleic acid molecules, the method comprising:
in a plurality of reaction sites of a reaction device comprising a plurality of field effect transistors corresponding to the plurality of reaction sites, distributing a reaction solution comprising at least two target molecules between the reaction sites (102), wherein at least two of the reaction sites contain a reagent to support amplification of the at least two target molecules, the at least two target molecules being chemically different from one another;
subjecting the reaction solution in the reaction sites to an amplification assay (106), wherein an amplified product of at least one of the target molecules is produced in at least some of the plurality of reaction sites;
detecting the presence of the amplified product by sensing a change in current or voltage using at least one of the field effect transistors (110);
based on detecting the presence of the amplified product, determining the presence of the at least one of the target molecule (112), wherein the plurality of reaction sites of the reaction device comprise a first subset of reaction sites (410) having a size that is configured to perform digital PCR and a second subset of reaction sites (412) having a size that is configured to perform quantitative PCR.

2. The method of claim 1, wherein the at least two target molecules comprise one or more of a DNA sequence, an RNA sequence, a gene, or an oligonucleotide.

3. The method of claim 1 or 2, wherein the at least two target molecules are specific to (a) a plurality of different organisms or viruses and/or (b) a plurality of different variants of a same organism or virus.

4. The method according to any one of claims 1-3, wherein (a) at least some of the plurality of reaction sites contain a reagent to support amplification of target molecules specific to a plurality of different organisms or viruses and/or (b) a subset of reaction sites of the plurality of reaction sites contain reagents to support amplification of one or more target molecules common to the different organisms or viruses.

5. The method according to any one of claims 1-4, wherein (a) at least some of the plurality of reaction sites contain a reagent to support amplification of target molecules specific to a plurality of different variants of an organism or virus and/or (b) a subset of reaction sites of the plurality of reaction sites contain a reagent to support amplification of one or more target molecules common to the different variants.

6. The method of claim 5, further comprising:
providing at least one group of reaction sites comprising a reagent to support amplification of a target molecule specific to at least one of a same organism or virus, or a same variant of a particular organism or virus.

7. The method of according to any one of claims 1-6, wherein a subset of the plurality of reaction sites contain a reagent to support amplification of a target molecule specific to at least one of a different organism or virus, or one of the different variant of the same organism or virus.

8. The method according to any one of claims 1-7, further comprising introducing a reaction solution into the reaction device so as to segregate the reaction solution.

9. A system (40) for detecting one or more target molecules of a reaction solution, the system comprising:
a reaction device (10) comprising a plurality of reaction sites (304) and one or more field effect transistors (308), at least two of the reaction sites configured to receive a reaction solution including at least two target molecules that are chemically different from one another;
a temperature controller (20) configured to control a temperature of at least one of the reaction sites or the reaction solution received by the at least one reaction site;
wherein at least some of the reaction sites are configured to receive a reagent supporting an amplification reaction of the at least two target molecules;
wherein, when the reaction solution in the at least some of the reaction sites is subjected to an amplification assay, an amplified product is produced in the at least some of the reaction sites; and
wherein the plurality of field effect transistors are configured to detect at least one of the amplified product,
wherein the plurality of reaction sites of the reaction device comprise a first subset of reaction sites (410) having a size that is configured to perform digital PCR and a second subset of reaction sites (412) having a size that is configured to perform quantitative PCR.

10. The system according to claim 9, wherein the at least two target molecules comprise one or more of a DNA sequence, an RNA sequence, a gene, an oligonucleotide, or an amino acid sequence.

11. The system according to any one of claims 9 -10, wherein the at least two target molecules are specific to different organisms or viruses and/or the at least two target molecules are specific to different variants of a same organism or virus.

12. The system according to any one of claims 9-11, wherein the reaction device is further configured to segregate the nucleic acid solution.

13. The system according to any one of claims 9-12, wherein the at least two target molecules are specific to (a) a plurality of different organisms or viruses and/or (b) a plurality of different variants of a same organism or virus.

14. The system according to any one of claims 9-13, wherein (a) at least some of the plurality of reaction sites contain a reagent to support amplification of target molecules specific to a plurality of different organisms or viruses and/or (b) a subset of reaction sites of the plurality of reaction sites contain reagents to support amplification of one or more target molecules common to the different organisms or viruses.

15. The system according to any one of claims 9-14, wherein (a) at least some of the plurality of reaction sites contain a reagent to support amplification of target molecules specific to a plurality of different variants of an organism or virus and/or (b) a subset of reaction sites of the plurality of reaction sites contain a reagent to support amplification of one or more target molecules common to the different variants.

## Patentansprüche

1. Verfahren zum Erkennen von Nukleinsäuremolekülen, wobei das Verfahren Folgendes umfasst:
in mehreren Reaktionsstellen einer Reaktionsvorrichtung, die mehrere Feldeffekttransistoren umfasst, die den mehreren Reaktionsstellen entsprechen, Verteilen einer Reaktionslösung, die wenigstens zwei Zielmoleküle umfasst, zwischen den Reaktionsstellen (102), wobei wenigstens zwei der Reaktionsstellen ein Reagens enthalten, um eine Amplifikation der wenigstens zwei Zielmoleküle zu unterstützen, wobei sich die wenigstens zwei Zielmoleküle chemisch voneinander unterscheiden;
Unterziehen der Reaktionslösung in den Reaktionsstellen einem Amplifikationsassay (106), wobei ein amplifiziertes Erzeugnis von wenigstens einem der Zielmoleküle in wenigstens einigen der mehreren Reaktionsstellen erzeugt wird;
Erkennen des Vorhandenseins des amplifizierten Erzeugnisses durch Erfassen einer Strom- oder Spannungsänderung unter Verwendung von wenigstens einem der Feldeffekttransistoren (110);
basierend auf dem Erkennen der Anwesenheit des amplifizierten Erzeugnisses, Bestimmen der Anwesenheit des wenigstens einen der Zielmoleküle (112), wobei die mehreren Reaktionsstellen der Reaktionsvorrichtung eine erste Teilmenge von Reaktionsstellen (410), die eine Größe aufweist, die konfiguriert ist, um eine digitale PCR durchzuführen, und eine zweite Teilmenge von Reaktionsstellen (412) umfassen, die eine Größe aufweist, die konfiguriert ist, um eine quantitative PCR durchzuführen.

2. Verfahren nach Anspruch 1, wobei die wenigstens zwei Zielmoleküle eine DNA-Sequenz, eine RNA-Sequenz, ein Gen und/oder ein Oligonukleotid umfassen.

3. Verfahren nach Anspruch 1 oder 2, wobei die wenigstens zwei Zielmoleküle für (a) mehrere unterschiedliche Organismen oder Viren und/oder (b) mehrere unterschiedliche Varianten eines gleichen Organismus oder Virus spezifisch sind.

4. Verfahren nach einem der Ansprüche 1-3, wobei (a) wenigstens einige der mehreren Reaktionsstellen ein Reagens enthalten, um die Amplifikation von Zielmolekülen zu unterstützen, die für mehrere unterschiedliche Organismen oder Viren spezifisch sind, und/oder (b) eine Teilmenge von Reaktionsstellen der mehreren Reaktionsstellen Reagenzien enthält, um die Amplifikation eines oder mehrerer Zielmoleküle zu unterstützen, die den unterschiedlichen Organismen oder Viren gemeinsam sind.

5. Verfahren nach einem der Ansprüche 1-4, wobei (a) wenigstens einige der mehreren Reaktionsstellen ein Reagens enthalten, um die Amplifikation von Zielmolekülen zu unterstützen, die für mehrere unterschiedliche Varianten eines Organismus oder Virus spezifisch sind, und/oder (b) eine Teilmenge von Reaktionsstellen der mehreren Reaktionsstellen ein Reagens enthält, um die Amplifikation eines oder mehrerer Zielmoleküle zu unterstützen, die den unterschiedlichen Varianten gemeinsam sind.

6. Verfahren nach Anspruch 5, das ferner Folgendes umfasst:
Bereitstellen von wenigstens einer Gruppe von Reaktionsstellen, die ein Reagens umfasst, um die Amplifikation eines Zielmoleküls zu unterstützen, das für einen Organismus oder Virus und/oder eine gleiche Variante eines speziellen Organismus oder Virus spezifisch ist.

7. Verfahren nach einem der Ansprüche 1-6, wobei eine Teilmenge der mehreren Reaktionsstellen ein Reagens enthält, um die Amplifikation eines Zielmoleküls zu unterstützen, das für einen unterschiedlichen Organismus oder Virus und/oder eine der unterschiedlichen Varianten des gleichen Organismus oder Virus spezifisch ist.

8. Verfahren nach einem der Ansprüche 1-7, das ferner ein Einführen einer Reaktionslösung in die Reaktionsvorrichtung umfasst, so dass die Reaktionslösung getrennt wird.

9. System (40) zum Erkennen eines oder mehrerer Zielmoleküle einer Reaktionslösung, wobei das System Folgendes umfasst:
eine Reaktionsvorrichtung (10), die mehrere Reaktionsstellen (304) und einen oder mehrere Feldeffekttransistoren (308) umfasst, wobei wenigstens zwei der Reaktionsstellen konfiguriert sind, um eine Reaktionslösung aufzunehmen, die wenigstens zwei Zielmoleküle beinhaltet, die chemisch unterschiedlich voneinander sind;
einen Temperaturregler (20), der konfiguriert ist, um eine Temperatur von der Reaktionsstelle und/oder der Reaktionslösung, die durch die wenigstens eine Reaktionsstelle aufgenommen wird, zu regeln;
wobei wenigstens einige der Reaktionsstellen konfiguriert sind, um ein Reagens aufzunehmen, das eine Amplifikationsreaktion der wenigstens zwei Zielmoleküle unterstützt;
wobei, wenn die Reaktionslösung an den wenigstens einigen der Reaktionsstellen einem Amplifikationsassay unterzogen wird, ein amplifiziertes Erzeugnis an den wenigstens einigen der Reaktionsstellen erzeugt wird; und
wobei die mehreren Feldeffekttransistoren konfiguriert sind, um wenigstens eines der amplifizierten Erzeugnisse zu erkennen,
wobei die mehreren Reaktionsstellen der Reaktionsvorrichtung eine erste Teilmenge von Reaktionsstellen (410), die eine Größe aufweisen, die konfiguriert ist, um eine digitale PCR durchzuführen, und eine zweite Teilmenge von Reaktionsstellen (412) umfassen, die eine Größe aufweisen, die konfiguriert ist, um eine quantitative PCR durchzuführen.

10. System nach Anspruch 9, wobei die wenigstens zwei Zielmoleküle eine DNA-Sequenz, eine RNA-Sequenz, ein Gen, ein Oligonukleotid und/oder eine Aminosäuresequenz umfassen.

11. System nach einem der Ansprüche 9-10, wobei die wenigstens zwei Zielmoleküle für unterschiedliche Organismen oder Viren spezifisch sind und/oder die wenigstens zwei Zielmoleküle für unterschiedliche Varianten eines gleichen Organismus oder Virus spezifisch sind.

12. System nach einem der Ansprüche 9-11, wobei die Reaktionsvorrichtung ferner konfiguriert ist, um die Nukleinsäurelösung zu trennen.

13. System nach einem der Ansprüche 9-12, wobei die wenigstens zwei Zielmoleküle für (a) mehrere unterschiedliche Organismen oder Viren und/oder (b) mehrere unterschiedliche Varianten eines gleichen Organismus oder Virus spezifisch sind.

14. System nach einem der Ansprüche 9-13, wobei (a) wenigstens einige der mehreren Reaktionsstellen ein Reagens enthalten, um die Amplifikation von Zielmolekülen zu unterstützen, die für mehrere unterschiedliche Organismen oder Viren spezifisch sind, und/oder (b) eine Teilmenge von Reaktionsstellen der mehreren Reaktionsstellen Reagenzien enthält, um die Amplifikation eines oder mehrerer Zielmoleküle zu unterstützen, die den unterschiedlichen Organismen oder Viren gemeinsam sind.

15. System nach einem der Ansprüche 9-14, wobei (a) wenigstens einige der mehreren Reaktionsstellen ein Reagens enthalten, um die Amplifikation von Zielmolekülen zu unterstützen, die für mehrere unterschiedliche Varianten eines Organismus oder Virus spezifisch sind, und/oder (b) eine Teilmenge von Reaktionsstellen der mehreren Reaktionsstellen ein Reagens enthält, um die Amplifikation eines oder mehrerer Zielmoleküle zu unterstützen, die den unterschiedlichen Varianten gemeinsam sind.

## Revendications

1. Procédé de détection de molécules d'acide nucléique, le procédé comprenant :
dans une pluralité de sites de réaction d'un dispositif de réaction comprenant une pluralité de transistors à effet de champ correspondant à la pluralité de sites de réaction, la distribution d'une solution de réaction comprenant au moins deux molécules cibles entre les sites de réaction (102), au moins deux des sites de réaction contenant un réactif pour supporter l'amplification des au moins deux molécules cibles, les au moins deux molécules cibles étant chimiquement différentes l'une de l'autre ;
la soumission de la solution de réaction dans les sites de réaction à un essai d'amplification (106), un produit amplifié d'au moins une des molécules cibles étant produit dans au moins certains de la pluralité de sites de réaction ;
la détection de la présence du produit amplifié en détectant un changement de courant ou de tension à l'aide d'au moins l'un des transistors à effet de champ (110) ;
sur la base de la détection de la présence du produit amplifié, la détermination de la présence d'au moins une des molécules cibles (112), la pluralité de sites de réaction du dispositif de réaction comprenant un premier sous-ensemble de sites de réaction (410) ayant une taille qui est conçue pour effectuer une PCR numérique et un second sous-ensemble de sites de réaction (412) ayant une taille qui est conçue pour effectuer une PCR quantitative.

2. Procédé selon la revendication 1, dans lequel les au moins deux molécules cibles comprennent une séquence d'ADN et/ou une séquence d'ARN et/ou un gène et/ou un oligonucléotide.

3. Procédé selon la revendication 1 ou 2, dans lequel les au moins deux molécules cibles sont spécifiques (a) d'une pluralité d'organismes ou virus différents et/ou (b) d'une pluralité de variants différents d'un même organisme ou virus.

4. Procédé selon l'une quelconque des revendications 1 à 3, dans lequel (a) au moins certains de la pluralité de sites de réaction contiennent un réactif pour supporter l'amplification de molécules cibles spécifiques à une pluralité d'organismes ou de virus différents et/ou (b) un sous-ensemble de sites de réaction de la pluralité de sites de réaction contient des réactifs pour supporter l'amplification d'une ou plusieurs molécules cibles communes aux différents organismes ou virus.

5. Procédé selon l'une quelconque des revendications 1 à 4, dans lequel (a) au moins certains de la pluralité de sites de réaction contiennent un réactif pour supporter l'amplification de molécules cibles spécifiques à une pluralité de variants différents d'un organisme ou d'un virus et/ou (b) un sous-ensemble de sites de réaction de la pluralité de sites de réaction contient un réactif pour supporter l'amplification d'une ou plusieurs molécules cibles communes aux différents variants.

6. Procédé selon la revendication 5, comprenant en outre :
la fourniture d'au moins un groupe de sites de réaction comprenant un réactif pour supporter l'amplification d'une molécule cible spécifique d'au moins l'un d'un même organisme ou virus, ou d'un même variant d'un organisme ou virus particulier.

7. Procédé selon l'une quelconque des revendications 1 à 6, dans lequel un sous-ensemble de la pluralité de sites de réaction contient un réactif pour supporter l'amplification d'une molécule cible spécifique d'au moins l'un d'un différent organisme ou virus, ou l'un des différents variants du même organisme ou virus.

8. Procédé selon l'une quelconque des revendications 1 à 7, comprenant en outre l'introduction d'une solution de réaction dans le dispositif de réaction de manière à séparer la solution de réaction.

9. Système (40) de détection d'une ou plusieurs molécules cibles d'une solution de réaction, le système comprenant :
un dispositif de réaction (10) comprenant une pluralité de sites de réaction (304) et un ou plusieurs transistors à effet de champ (308), au moins deux des sites de réaction conçus pour recevoir une solution de réaction comportant au moins deux molécules cibles qui sont chimiquement différentes l'une de l'autre ;
un régulateur de température (20) configuré pour réguler une température d'au moins l'un des sites de réaction ou de la solution de réaction reçue par l'au moins un site de réaction ;
au moins certains des sites de réaction étant conçus pour recevoir un réactif supportant une réaction d'amplification des au moins deux molécules cibles ;
lorsque la solution de réaction dans au moins certains des sites de réaction est soumise à un essai d'amplification, un produit amplifié étant produit dans au moins certains des sites de réaction ; et
la pluralité de transistors à effet de champ étant configurés pour détecter au moins l'un des produits amplifiés,
la pluralité de sites de réaction du dispositif de réaction comprenant un premier sous-ensemble de sites de réaction (410) ayant une taille qui est conçue pour effectuer une PCR numérique et un second sous-ensemble de sites de réaction (412) ayant une taille qui est conçue pour effectuer une PCR quantitative.

10. Système selon la revendication 9, dans lequel les au moins deux molécules cibles comprennent une séquence d'ADN, une séquence d'ARN et/ou un gène et/ou un oligonucléotide et/ou une séquence d'acides aminés.

11. Système selon l'une quelconque des revendications 9 à 10, dans lequel les au moins deux molécules cibles sont spécifiques de différents organismes ou virus et/ou les au moins deux molécules cibles sont spécifiques de variants différents d'un même organisme ou virus.

12. Système selon l'une quelconque des revendications 9 à 11, dans lequel le dispositif de réaction est en outre configuré pour séparer la solution d'acide nucléique.

13. Système selon l'une quelconque des revendications 9 à 12, dans lequel les au moins deux molécules cibles sont spécifiques (a) d'une pluralité d'organismes ou virus différents et/ou (b) d'une pluralité de variants différents d'un même organisme ou virus.

14. Système selon l'une quelconque des revendications 9 à 13, dans lequel (a) au moins certains de la pluralité de sites de réaction contiennent un réactif pour supporter l'amplification de molécules cibles spécifiques à une pluralité d'organismes ou de virus différents et/ou (b) un sous-ensemble de sites de réaction de la pluralité de sites de réaction contient des réactifs pour supporter l'amplification d'une ou plusieurs molécules cibles communes aux différents organismes ou virus.

15. Système selon l'une quelconque des revendications 9 à 14, dans lequel (a) au moins certains de la pluralité de sites de réaction contiennent un réactif pour supporter l'amplification de molécules cibles spécifiques à une pluralité de variants différents d'un organisme ou d'un virus et/ou (b) un sous-ensemble de sites de réaction de la pluralité de sites de réaction contient un réactif pour supporter l'amplification d'une ou plusieurs molécules cibles communes aux différents variants.
